(19) Europäisches Patentamt
European Patent Office
Office européen des brevets

(11) Publication number: **0 518 428 A2**

# (12) EUROPEAN PATENT APPLICATION

(21) Application number: **92201645.6**

(22) Date of filing: **05.06.92**

(51) Int. Cl.[5]: **C08F 4/46**, C08F 36/04, C07C 43/305, C07C 43/32

(30) Priority: **10.06.91 GB 9112419**

(43) Date of publication of application: **16.12.92 Bulletin 92/51**

(84) Designated Contracting States: **BE DE ES FR GB IT NL**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Koppes, Margaretha Johanna Caecilia Maria**
**Badhuisweg 3**
**NL-1031 CM Amsterdam(NL)**
Inventor: **van der Arend, Johannes Cornelis Maria**
**Badhuisweg 3**
**NL-1031 CM Amsterdam(NL)**

## (54) Process for polymer preparation.

(57) A process for the preparation of structure-modified conjugated diene based polymers by polymerizing at least a conjugated diene in an inert hydrocarbon diluent and in the presence of an organo-alkali metal initiator and a specified compound of general formula

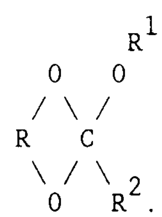

EP 0 518 428 A2

The present invention relates to a process for the preparation of polymers based on at least one or more conjugated dienes, in the presence of an organo-lithium initiator and more in particular to the preparation of such polymers in the presence of a structure modifier, and to the polymers thus prepared.

The preparation of conjugated diene based polymers in the presence of an organo-lithium initiator and a structure modifier is known and is generally conducted in a reaction medium comprising one or more hydrocarbon solvent compounds such as for example linear, branched or cycloaliphatic compounds, or aromatic compounds.

The conjugated diene homo- and copolymers prepared by a process as described hereinbefore are generally characterized in that on average up to about 60% or more of the conjugated diene units have polymerized via a 1,2- or 3,4-addition. The percentage of diene units in a polymer which has reacted via a 1,2- or 3,4- addition will hereinafter be referred to as the vinyl content. The vinyl content of the corresponding polymers prepared in the absence of a structure modifier would on the other hand be on average in the range of up to about 10%.

Many of the structure modifiers which have been proposed in the past, such as the widely recommended diethyleneglycol dimethyl ether, frequently have a tendency to terminate the polymerization reaction prematurely and/or to disturb the coupling reaction of the living polymer, when used. As a result of these effects the polymer obtained exhibits a wider molecular weight distribution than would be obtainable in the absence of such a modifier, while simultaneously a lower degree of monomer conversion and/or coupling efficiency may be obtained. Without wishing to be bound by any theory or theoretical considerations it is conceivable that the cause of these blemishes may be found in the sensitivity of the anionic polymerization to the presence of protic and other polar compounds which are able to react with the living polymer, and which compounds will hereinafter be referred to as polymer-reactive compounds. Such polymer-reactive compounds may be formed e.g. in situ as a result of insufficient stability of the polar structure modifier during polymerization. This has been known to occur with some of the alkyl ethers of mono- and polyethylene glycol, especially the methyl ethers. A further potential source for the formation of polymer-reactive compounds is the steam distillation step, which is generally applied to separate off the solvent fraction upon completion of the polymer preparation, and which solvent fraction will subsequently be reused in further polymerization processes. Under the conditions of steam distillation some of the polar modifiers may be converted to one or more polymer-reactive compounds. When such a polymer-reactive compound has approximately the same boiling point as the hydrocarbyl solvent to be removed, and is moreover substantially water-immiscible, it will end up in the same fraction as the hydrocarbyl solvent. Such a contaminated hydrocarbyl solvent will thus require purification to remove the polymer-reactive compounds before being recycled to the polymer preparation section, which is of course economically unattractive. A group of structure modifiers which may give rise to the formation of polymer-reactive compounds during steam distillation and which compounds include potential contaminants for the hydrocarbon solvent fraction to be recycled, are the orthoesters, as described e.g. in U.S. Patent Specification No. 3,580,895.

A group of structure modifiers which are considerably more stable during polymerization and/or steam distillation, are the conventional cyclic ethers. Examples of such cyclic ethers include tetrahydrofuran, which compound is however known to be only effective when present in a high concentration and is moreover not a very attractive compound for use on an industrial scale; as well as cyclic ethers having an -O-C-O-group in the ringstructure. However, in U.S. Patent Specification No. 3,288,872 it is stated that all cyclic ethers having an -O-CO-group in a ringstructure inhibit the organo-alkali metal initiated polymerization of conjugated diene so excessively, that their use is impractical.

Hence it can be concluded that there is considerable need for an improved process for preparing structure-modified conjugated diene based polymers, i.e. polymers obtained via an anionic polymerization process having a vinyl content which is comparable with that of polymers prepared by using one or more beforementioned structure modifiers and which polymers are substantially free of the beforementioned polymer-reactive compounds. Therefore the problem underlying the present invention is to develop a process for the preparation of said structure modified conjugated diene polymers, which does not suffer from the presence or formation of polymer-reactive compounds as mentioned hereinbefore.

As a result of extensive research and experimentation it was surprisingly found that the requirements as set out hereinbefore could be met by conducting the preparation of said conjugated diene polymers via an anionic polymerization process in the presence of selected substituted dioxolane type compounds.

Accordingly the invention provides a process for the preparation of structure-modified conjugated diene based polymers, by polymerizing at least a conjugated diene in an inert hydrocarbon diluent and in the presence of an organo-alkali metal initiator compound and a compound of general formula

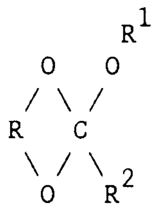

(I)

wherein R is an alkylene bridging group having 2 to 4 carbon atoms in the bridge, $R^1$ is an alkyl group having from 1 to 10 carbon atoms, and $R^2$ is a hydrocarbyl group or H.

A major advantage of the process of the present invention is that the products obtained are substantially free from polymer-reactive compounds, as described hereinbefore.

Preferably R is an alkylene group having 4 carbon atoms in the bridge, which bridge may furthermore carry one or more inert substituents, i.e. substituents which are inert under the conditions of polymer preparation, e.g. which do not react with the living polymer species, such as for example alkyl groups.

$R^1$ is preferably a lower alkyl group having not more than 4 carbon atoms, and is more preferably an ethyl group. The hydrocarbyl group $R^2$ is conveniently an alkyl or aryl group which may optionally carry one or more inert substituents, wherein the term inert has the same meaning as hereinbefore. Preferably $R^2$ represents a methyl group. The preferred compound of general formula I is 2-ethoxy-2-methyl-1,3-dioxacycloheptane. The compound of general formula I wherein R is 2-ethylethylene, $R^1$ is ethyl and $R^2$ is methyl, i.e. 2-ethoxy-2-methyl-4-ethyl-1,3-dioxolane is novel, and forms an other aspect of the present invention.

The structure modifier of general formula I will preferably be employed in an amount in a range of from 10 to 1000 ppm and more preferably in a range of from 50 to 850 ppm calculated on the total weight of monomer and hydrocarbon diluent. The hydrocarbon diluents which may conveniently be employed in the process of the present invention include, aliphatic, cycloaliphatic and aromatic hydrocarbons having 4-10 carbon atoms, or mixtures thereof, as exemplified by n-hexane, n-heptane, cyclopentane, cyclohexane, 2,2,4-trimethylpentane, benzene and toluene or mixtures thereof. Cyclohexane and cyclopentane are the preferred diluents for use in the process of the present invention. The hydrocarbon diluent will generally be employed in an amount of at least 100 parts by weight of diluent, and more preferably in an amount in the range of from 200-1500 parts by weight per 100 parts by weight of monomer.

The organo-alkali metal initiator compound for use in the process of the present invention is generally a hydrocarbyllithium compound having one or more lithium atoms per molecule. Preferably the hydrocarbyllithium compound is a monolithium compound. The hydrocarbyl group will generally be an aliphatic, cycloaliphatic or aromatic hydrocarbyl group having from 1-20 carbon atoms, such as for example methyl, n-butyl, sec-butyl, phenyl and naphthyl; sec-butyllithium being the preferred hydrocarbyllithium initiator.

The conjugated diene monomers which may be employed in the process of the present invention will generally have from 4-8 carbon atoms and include compounds such as 1,3-butadiene, isoprene, piperylene and 2,3-dimethyl-1,3-butadiene, 1,3-butadiene and isoprene being preferred conjugated dienes.

The polymers which may be prepared according to the process of the present invention include conjugated diene homopolymers such as for example polybutadiene and polyisoprene, conjugated diene copolymers, such as for example random and block copolymers comprising at least two conjugated dienes, such as for example 1,3-butadiene and isoprene, or copolymers comprising at least one conjugated diene and at least one anionically polymerizable compound. A preferred class of such polymerizable compounds are monoalkenyl aromatic hydrocarbons, and includes styrene, ring-substituted styrene such a 3-methylstyrene, 4-n-propylstyrene, 4-cyclohexylstyrene, 4-decylstyrene, 4-p-tolylstyrene, $\alpha$-methylstyrene and vinylnaphthalene. Styrene is the preferred monoalkenyl aromatic hydrocarbon. Examples of copolymers comprising at least a conjugated diene and at least a monoalkylene aromatic compound, include random copolymers and block copolymers of at least a conjugated diene and at least a monoalkenyl aromatic compound, and in particular block copolymers, e.g. di-, tri- and polyblock copolymers of butadiene and styrene or isoprene and styrene, as well as tapered block copolymers based on monomer combinations as described hereinbefore. The block copolymers include linear and branched block copolymers. Examples of preferred conjugated diene based triblock copolymers include BIB; SBS and SIS triblock copolymers wherein B, I and S respectively represent a poly-1,3-butadiene, a polyisoprene and a polystyrene block. The linear block copolymers may be prepared by sequential polymerization techniques or by a combination of sequential polymerization and coupling techniques employing a difunctional coupling agent. The branched block copolymers may be prepared by a combination of sequential polymerization and coupling techniques employing a coupling agent having a coupling functionality ≧ 3, or only by sequential

polymerization when an organolithium compound is employed having at least 3 lithium atoms per molecule.

Random conjugated diene- monoalkylene aromatic compound based copolymers will generally be prepared by conducting the polymerization in the presence of both the conjugated diene and the monoalkylene aromatic compound, which may be conducted batchwise, semi-batchwise or continuously.

The polymerization will generally be conducted at a temperature in the range of from 10 to 100 °C and preferably in the range of from 30 to 80 °C.

When the polymerization reaction has progressed to the desired degree of conversion the reaction may be terminated by methods generally known in the art. Alternatively, the living polymer may be coupled by means of a di- or multifunctional coupling agent, depending on the type of coupled polymer to be prepared, i.e. a linear or a branched polymer. Dibromoethane is a well-known difunctional coupling agent, while diethyl adipate, $CH_3SiCl_3$ and $SiCl_4$ are commonly used as multifunctional coupling agents. The amount of coupling agent to be employed is largely determined by its functionality; difunctional coupling agents generally being employed in a range of from 0,25 to 0,75 mol per mol of alkali metal of the initiator, while the amount of polyfunctional coupling agent will generally be in the range of from 0,1 to 0,5 mol per mol of alkali metal of the initiator.

The vinyl content of the polymers prepared in the presence of a structure modifier of general formula I may vary over wide ranges provided the alkylene bridging group R in general formula I has 4 carbon atoms in the bridge. When on the other hand the bridging group R has only 2 or 3 C atoms in the bridge, the activity of the structure modifier is considerably lower, and such structure modifiers will generally also require a higher minimum concentration to be effective and or a lower temperature of polymerization. Although in general preference will be given to structure modifiers of general formula I, wherein R has 4 carbon atoms in the bridge in view of the considerably higher vinyl content obtainable therewith, the corresponding structure modifiers having only 2 or 3 carbon atoms in the bridge may be advantageously used in the preparation of structure modified polymers having a relatively low vinyl content e.g. in the range of from ≧ 10 to 25% and wherein an overshoot of the target vinyl content is unacceptable.

In addition to having structure modifying properties, the compounds of formula I were also found to have the well-known randomnizing effect of Lewis bases in copolymerization reactions e.g. in the copolymerization of styrene and 1,3-butadiene, which phenomenon has been described in e.g. U.S. Patent Specification No. 4,530,985.

The polymers prepared by the process of the present invention may conveniently be employed as such, or optionally in blends with other polymers, in a wide range of applications such as in adhesives, footwear compositions, and U.V. curable compositions. Furthermore a number of such polymers may advantageously be selectively hydrogenated by known processes to obtain polymers substantially free from ethylenically unsaturated groups.

A further aspect of the present invention relates to the preparation of the novel compound of general formula I, i.e. 2-ethoxy-2-methyl-4-ethyl-1,3-dioxolane. Said compound may conveniently be prepared by a process comprising the reaction of triethylorthoacetate with 1,2-butanediol in the presence of an acidic catalyst, e.g. p-toluene sulfonic acid, at elevated temperature, e.g. 110 °C, with removal of the coproduced ethanol.

The invention will be further illustrated by the following examples without restricting the scope of the invention, and for which the following information is provided.

The structure modifiers used in Examples II-XVII hereinafter were prepared by transesterification of acyclic orthoesters with the appropriate diol as described by R.H. De Wolfe in "Carboxylic Ortho Acid Derivatives", Academic Press 1970 and by R.P. Narain and A.J. Kawr, Indian J. Chem. Sect. B, 17 B, 189-91, and as further exemplified in Example I hereinafter.

## Example I

Preparation of 2-ethoxy-2-methyl-4-ethyl-1,3-dioxolane.

A mixture of 16.2 g (0.1 mol) triethylorthoacetate and 9.5 g (0.105 mol) 1,2-butanediol and a catalytic amount of p-toluene sulfonic acid (0.005 g) was heated at 110 °C in a reaction flask equipped with a condensor and a Dean-Stark trap. After the collection of more than 10 ml of ethanol (ca. 2 hr), the residue was purified by distillation. Yield: 7.2 g (45%) of a colourless liquid (b.p.: 168 °C). $^1H$ NMR ($CDCl_3$, ppm): 0,9(dt, J = 7 Hz, $CCH_2CH_3$), 1,2(t, J = 7 Hz, 3H, $OCH_2CH_3$), 1,6(s, 3H, $CCH_3$), 1,7(m, 2H, $CCH_2CH_3$), 3,5-3,7(m, 3H), 4,0-4,3(m, 2H).

## Examples II-XI

The experiments were conducted in a 500 ml glass serum bottle into which were introduced 100 ml cyclohexane,
12 g 1,3-butadiene and a modifier of a type and in an amount as indicated in Table 1 hereinafter. After homogenizing the bottle contents they were heated to 50 °C in an oil bath. Subsequently 0.5 mmol of sec-butyllithium were introduced and mixed with the reactor contents, which were kept at 50 °C for 40 minutes.

The polymerization was terminated by injecting 2 ml of ethanol in the polymer cement, and containing 0.05 g of 2,6-tert-butyl-4-methylphenolas polymer stabilizer. The polymer was isolated by removing the volatile compounds by steam distillation, and the vinyl content was determined by infrared spectroscopy. The results are given in Table 1.

Table 1

| Example No. | Compound for formula I: R | $R^1$ | $R^2$ | Vinyl content %mol as function of concentration (ppm): 100 | 200 | 400 | 800 |
|---|---|---|---|---|---|---|---|
| II | Ethylene | Ethyl | H | 9 | 15 | 19 | 26 |
| III | Ethylene | " | Methyl | 13 | 16 | 19 | 26 |
| IV | Methylethylene | " | " | 12 | 14 | 19 | 26 |
| V | Ethylethylene | " | " | 12 | 14 | 18 | 24 |
| VI | Ethylene | " | Phenyl | 10 | 12 | 14 | 18 |
| VII | Trimethylene | " | Methyl | 10 | 11 | 13 | 16 |
| VIII | 1-Methyltrimethylene | " | " | 10 | 11 | 12 | 15 |
| IX | Tetramethylene | " | " | 42 | 46 | 53 | 59 |
| X | Ethylene | Methyl | Methyl | 10 | 11 | 13 | 20 |
| XI | Ethylene | n-Butyl | Methyl | 9 | 11 | 14 | 19 |

Example XII

The procedure of Example IX was repeated, i.e. using 2-ethoxy-2-methyl-1,3-dioxacycloheptane as the modifier but conducting the polymerization at a temperature of 10, 35, 40, 50 and 60 °C, instead of only at 50 °C. The resulting vinyl contents have been collected in Table 2.

Table 2

| Temp. °C | Vinyl content (%mol) as function of the modifier content (ppm) | | | |
|---|---|---|---|---|
| | 100 | 200 | 300 | 400 |
| 10 | 58.8 | 59.2 | 60.2 | 61.5 |
| 35 | 41.1 | 51.1 | 56.7 | 60.9 |
| 40 | 35.9 | 46.9 | 54.0 | 58.3 |
| 50 | 36.5 | 42.5 | 49.2 | 55.8 |
| 60 | 22.9 | 32.5 | 42.0 | 43.1 |

Example XIII

Preparation of a coupled styrene-butadiene branched copolymer.

In a 500 ml $N_2$-purged serum bottle 9.5 g styrene and 22.18 g 1,3-butadiene were polymerized in the presence of 250 ml cyclohexane, 0.4 mmol sec-butyllithium and 450 ppm of 2-ethoxy-2-methyl-1,3-dioxepane calculated on monomer and solvent at 50 °C for 2.5 h. Subsequently diethyladipate was added in a sec-butyllithium to diethyladipate molar ratio 4, and heating maintained for another 30 min. The polymer was analysed before and after coupling by means of gel permeation chromatography in order to determine the peak molecular weight before and after coupling, and the coupling efficiency. The vinyl content and the amount of styrene incorporated in the copolymer were determined by infrared spectroscopy, which provided the following data.

| | | |
|---|---|---|
| Peak molecular weight x$10^{-3}$ | before coupling | 91 |
| " " " " | after " | 329 |
| " " " " | " " | 259 |
| " " " " | " " | 181, |

coupling efficiency: 61%; vinyl content: 40 %m; and styrene incorporated 30 %m.

## Examples XIV-XVI

Preparation of styrene-butadiene copolymers.

In a 500 ml $N_2$-purged serum bottle styrene and butadiene were co-polymerised in 250 ml cyclohexane using sec-butyllithium as initiator and varying amounts of 2-ethyl-2-methyl-1,3-dioxepane. The polymerization was conducted for a short period 1.75 min. at 60 °C, in order to show the randomnizing effect of the modifier on the polymerization. The reaction was terminated by the addition of ethanol. The polymer so obtained was subjected to infrared spectroscopy in order to determine the vinyl content and the amount of styrene incorporated in the polymer. Further data and results are given in Table 3.

## Example XVII

Preparation of a styrene-butadiene-styrene block copolymer.

A 10 l stainless steel reactor was charged with 6 l cyclohexane and 295 g styrene, and heated to 60 °C. The polymerization was initiated by the introduction of 26 mmol sec-butyllithium; heating was continued until the polymerization had been completed (within 1 hour). Subsequently 3 ml of 2-ethoxy-2-methyl-1,3-dioxacycloheptane was added, which was followed by the gradual addition in 10 minutes of 705 g butadiene. Heating was continued to achieve a complete butadiene conversion (within 2 hours). Next 13 mmol dibromoethane were added and coupling of the living polymer species was allowed to proceed for 0.5 hours. Finally the polymer was isolated by steam coagulation followed by drying in an oven (50 °C).

The polymer was analyzed following the procedure as described in Example XIII, which provided the following data.

| | | |
|---|---|---|
| Peak molecular weight x$10^{-3}$ | before coupling | 49.4 |
| " " " " | after coupling | 103.3. |

Coupling efficiency: 81%; vinyl content: 56% and styrene incorporated 29.5%.

Table 3

| Example | Styrene | 1,3-Butadiene | S-Buli | Modifier | Conversion | Vinyl content* | Styrene content* |
|---|---|---|---|---|---|---|---|
| | (g) | (g) | (mmol) | (ppm) | (%) | (%m) | (%m) |
| XIV | 9.70 | 22.64 | 0.50 | 100 | 11.1 | 20.5 | 7.9 |
| XV | 8.73 | 20.36 | 0.50 | 350 | 7.9 | 38.5 | 16.7 |
| XVI | 9.21 | 21.49 | 0.99 | 600 | 19.8 | 41.9 | 18.5 |

* In the absence of the modifier, the styrene content was ≈ 2.5 %m.

## Claims

**1.** A process for the preparation of structure-modified conjugated diene based polymers by polymerizing at least a conjugated diene in an inert hydrocarbon diluent and in the presence of an organo-alkali metal initiator compound and a compound of general formula

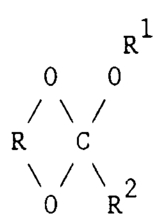

(I)

wherein R is an alkylene bridging group having 2 to 4 carbon atoms in the bridge, $R^1$ is an alkyl group

having from 1 to 10 carbon atoms, and $R^2$ is a hydrocarbyl group or H.

**2.** A process as claimed in claim 1, wherein R has 4 carbon atoms in the bridge.

**3.** A process as claimed in claim 1 or 2, wherein $R^1$ is a lower alkyl group having not more than 4 carbon atoms.

**4.** A process as claimed in any one of claims 1-3, wherein $R^1$ is ethyl.

**5.** A process as claimed in any one of claims 1-4, wherein $R^2$ represents a methyl group.

**6.** A process as claimed in claim 1, wherein the compound of formula I is 2-ethoxy-2-methyl-1,3-dioxacycloheptane.

**7.** A process as claimed in any one of claims 1-6, wherein the compound of formula I is employed in an amount in the range of from 10 to 1000 ppm and preferably from 50 to 850 ppm, calculated on total weight of monomer and hydrocarbon diluent.

**8.** A process as claimed in any one of claims 1-7, wherein the hydrocarbon diluent is cyclohexane.

**9.** A process as claimed in any one of claims 1-8, wherein the organo-alkali metal initiator is a hydrocarbyllithium compound.

**10.** A process as claimed in claim 9, wherein the hydrocarbyllithium compound is sec-butyllithium.

**11.** A process as claimed in any one of claims 1-10, wherein the conjugated diene is 1,3-butadiene or isoprene.

**12.** A process as claimed in any one of claims 1-11, wherein in addition to the at least one conjugated diene at least one monoalkylene aromatic compound is present as monomer.

**13.** A process as claimed in claim 12, wherein the monoalkylene aromatic compound is styrene.

**14.** A process as claimed in any one of claims 1-13, wherein the polymer is a triblock copolymer selective from the group consisting of BIB, SBS and SIS as defined hereinbefore.

**15.** 2-Ethoxy-2-methyl-4-ethyl-1,3-dioxane.

**16.** A process for the preparation for the compound as claimed in claim 15, which comprises reacting triethylorthoacetate with 1,2-butanediol in the presence of an acidic catalyst at elevated temperature with removal of the coproduced ethanol.

**17.** A conjugated diene based polymer whenever prepared according to a process as claimed in any one of claims 1-14.